# EUROPÄISCHE PATENTSCHRIFT

(11) **EP 0 413 006 B1**
(45) Veröffentlichungstag und Bekanntmachung des Hinweises auf die Patenterteilung: **19.10.1994**
(21) Anmeldenummer: 90902143.8
(22) Anmeldetag: 29.01.1990
(51) Int. Cl.: C07C 235/06, C07D 295/18, C07D 265/30, A61K 31/16, A61K 31/40, A61K 31/535

(54) **HYDROXYALKANCARBONSÄURE-DERIVATE UND DEREN VERWENDUNG**
HYDROXYALKANE CARBOXYLIC ACID DERIVATES AND THEIR USE
DERIVES D'ACIDE CARBOXYLIQUE D'HYDROXYALKANE ET LEUR EMPLOI

(30) Priorität: 17.02.1989 DE 3905325
(43) Veröffentlichungstag der Anmeldung: 20.02.1991
(60) Teilanmeldung: 93250197.6
(73) Patentinhaber: SCHERING AKTIENGESELLSCHAFT, 13342 Berlin (DE)
(72) Erfinder: EARNSHAW, Christopher, Cambridge CB4 1RP (GB); KIRSCH, Gerald, D-1000 Berlin 33 (DE); RACH, Petra, D-1000 Berlin 65 (DE); THIEROFF-EKERDT, Ruth, D-1000 Berlin 28 (DE); TÖPERT, Michael, D-1000 Berlin 28 (DE)
(86) Internationale Anmeldenummer: DE9000057
(87) Internationale Veröffentlichungsnummer: WO9009373

(56) Entgegenhaltungen:
- US-A- 2 372 797
- US-A- 2 936 325
- CHEMICAL ABSTRACTS, Band 104, Nr. 17, 28. April 1986, Columbus, Ohio, US; A. P. TULLOCH: "Mass spectra of pyrrolidides of oxo, hydroxy and trimethylsilyloxy octadecanoic acids", S. 625, Zusammenfassung Nr. 148126g
- CHEMICAL ABSTRACTS, Band 83, Nr. 14, 6. Oktober 1975, Columbus, Ohio, US; G. CZICHOCKI et al: "Anion-active surfactants from 10-undecenoic acid", S. 207, Zusammenfassung Nr. 117596d
- JOURNAL OF CHEMICAL SOCIETY C, Dezember 1968; D. F. JONES et al: "Microbiological oxidation of long-chain aliphatic compounds. Part IV. Alkane derivatives having polar terminal groups", S. 2821-2827

## Beschreibung

Die Erfindung betrifft die Verwendung von Hydroxyalkancarbonsäure-Derivaten der allgemeinen Formel I 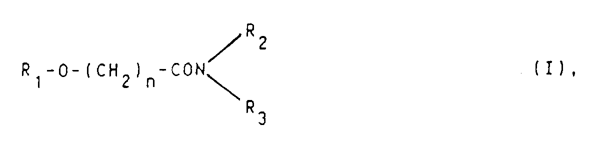
worin
- n: die Ziffern 7 bis 18 bedeutet,
- R₁: ein Wasserstoffatom oder eine Acylgruppe mit maximal 16 Kohlenstoffatomen darstellt und
- R₂: und R₃ jeweils Alkylgruppen mit maximal 8 Kohlenstoffatomen oder gemeinsam eine gegebenenfalls durch ein Sauerstoffatom oder ein Stickstoffatom unterbrochene Alkylengruppe mit 4 bis 8 Kohlenstoffatomen bedeutet, zu Herstellung eines Arzneimittels für die lokale Behandlung von Erkrankungen der Haut und Schleimhaut und einige zuvor unbekannte Hydroxyalkancarbonsäure-Derivate dieser Formel.

In der Publikation von G. Czichocki et. al (Tenside Detergens 11, 1974 p 298-305) sind das 11-Hydroxyundecansäure-diethylamid und das 11-Hydroxyundecansäuredibutylamid vorbeschrieben und in der Publikation von D.F. Jones et. al (J. Chem. Soc. (C), 1968, p 2821-2827) wird das 16-Hydroxyhexadecansäure-dimethylamid erwähnt. Hinweise über die pharmakologische Wirksamkeit dieser Substanzen findet man in der Literatur nicht.

Auch von anderen strukturanalogen Hydroxyalkancarbonsäureamiden, wie beispielsweise von Carbonsäuren mit bis zu 8 Kohlenstoffatomen (US-A 2,936,325) oder solchen von Carbonsäuren des Ricinusöls (US-A 2,372,797) ist nicht bekannt, daß sie pharmakologisch wirksam sind.

Es ist bekannt, daß zahlreiche Erkrankungen der Haut unter anderem auch durch Verhornungsstörungen derselben verursacht werden, die durch eine vermehrte Bildung und/oder eine verminderte Abschilferung von Hornzellen zustande kommt.

So ist nach heutigem Wissen an der Entstehung der Acne vulgaris und verwandter Erkrankungen des Talgdrüsenfollikels, wie zum Beispiel der Acne estivalis und der Acne venenata neben einer erhöhten Sebumproduktion und einer Besiedelung des Follikellumens mit Propionibacterium acnes auch eine Verhornungsstörung des Follikelepithels beteiligt.

Ichthyosiforme Dermatosen und follikuläre Hyperkeratosen sind Verhornungsstörungen der Haut. Psoriasis, parapsoriatische Erkrankungen der Haut und lichinoide Hauterkrankungen sind ebenfalls gekennzeichnet durch eine Verhornungsstörung und zusätzlich durch entzündliche Prozesse, sowie eine vermehrte Durchblutung und Zellinfiltrate.

Bei Warzen und Dermatomykosen ist die Verhornungsstörung neben der mikrobiologischen Komponente für den Krankheitsverlauf von Bedeutung.

Derartige mit Verhornungsstörungen verbundenen Erkrankungen der Haut werden nach dem bekannten Stand der Technik mit Keratinolytika, wie Harnstoff, Salicylsäure oder Benzoylperoxid mit Glucocortikoiden, Retinoiden und im Falle der Aknetherapie mit lokal oder systemisch verabreichten Antibiotika behandelt. Der Nutzen dieser Therapeutika ist entweder aufgrund ihrer begrenzten Wirksamkeit, wie bei den Keratolytika, oder wegen der lokaltoxischen sowie systemischen Nebenwirkungen bis hin zur Teratogenität der Retinoide, eingeschränkt. Aus diesen Grunde sind die Therapiemöglichkeiten von Erkrankungen, die mit Verhornungsstörungen der Haut einhergehen, bislang unbefriedigend.

Es wurde nun gefunden, daß die Hydroxyalkancarbonsäure-Derivate der allgemeinen Formel I gemäß Patentanspruch 1 überraschenderweise eine ausgeprägte antiproliferative Wirkung auf Keratinocyten besitzen, so daß zu erwarten ist, daß Arzneimittel, die diese Wirkstoffe enthalten, zur lokalen Behandlung von mit Verhornungsstörungen und/oder Hyperproliferation der Epidermis verbundenen Erkrankungen der Haut sehr geeignet sind. Diese Hydroxyalkancarbonsäure-Derivate zeigen weiterhin eine ausgeprägte komedolytische, antiinflammatorische und antimikrobielle Wirksamkeit und haben den Vorzug einer sehr geringen Toxizität, so daß diese Wirkstoffe enthaltende Arzneimittel zur lokalen Behandlung zahlreicher Erkrankungen der Haut und Schleimhaut Anwendung finden können.

Die antiproliferative Wirkung auf Keratinocyten wurde in vitro wie folgt ermittelt:
Primärkulturen neonataler muriner Keratinocyten werden in serumfreien Medium 28 Stunden lang mit den Prüfsubstanzen und terminal 4 Stunden lang mit Methyl-3H Thymidin inkubiert.

Der 3H-Thymidineinbau in die DNA der Keratinocyten dient als Maß für die Proliferationsrate derselben. In diesem Test werden folgende Ergebnisse erzielt:

**Tabelle 1**

| Nr. | Testsubstanz | mol/l | % Hemmung* |
|---|---|---|---|
| I | 11-Hydroxy-undecansäuredimethylamid | 10⁻⁵ | 30 % |
| | | 10⁻⁴ | 70 % |
| II | 12-Hydroxy-dodecansäuredimethylamid | 10⁻⁵ | 20 % |
| | | 10⁻⁴ | 85 % |
| III | 13-Hydroxy-tridecansäure-dimethylamid | 10⁻⁵ | 70 % |
| | | 10⁻⁴ | 95 % |
| IV | 14-Hydroxy-tetradecansäure-dimethylamid | 10⁻⁵ | 85 % |
| | | 10⁻⁴ | 90 % |
| V | 13-Hydroxy-tridecansäuredipropylamid | 10⁻⁵ | 91 % |
| | | 10⁻⁴ | 92 % |
| VI | 13-Hydroxy-tridecansäuretetramethylenamid | 10⁻⁵ | 24 % |
| | | 10⁻⁴ | 92 % |
| VII | 13-Hydroxy-tridecansäure(2,4-dimethyl-3-oxa-pentamethylen)-amid | 10⁻⁵ | 22 % |
| | | 10⁻⁴ | 62 % |
| VIII | 13-Acetoxy-tridecansäuredimethylamid | 10⁻⁵ | 32 % |
| | | 10⁻⁴ | 89 % |
| IX | 13-(2,2-Dimethylpropionyloxy)-tridecansäure-dimethylamid | 10⁻⁵ | 86 % |
| | | 10⁻⁴ | 93 % |

| | | | |
|---|---|---|---|
| * bezogen auf die Vehikel-Kontrolle | | | |

Die komedolytische Wirksamkeit der Hydroxyalkancarbonsäure-Derivate wird ermittelt, indem man an der Innenseite von Kaninchenohren durch topische Applikation von 200 µl Tetradecan nach einer 10tägigen Behandlung komedonenähnliche Veränderungen der Haarfollikel mit Epithelhyperplasie und Hornzellenretentien induziert und auf die so behandelten Stellen 11 Tage lang täglich jeweils 200 µl einer 5 %igen ethanolischen Lösung der Prüfsubstanz topisch appliziert.

Dann werden die behandelten Areale ausgestanzt und die Verkleinerung der erweiterten Follikellumina ermittelt, welche als Maß für die komedolytische Aktivität gilt.

In diesem Test zeigt eine 5 %ige Lösung von N,N-Dimethyl-13-hydroxy-tridecansäureamid eine 44,5 %ige Hemmung bezogen auf die Vehikelkontrolle.

Zur Bestimmung der antimikrobiellen Wirksamkeit werden unter den üblichen Bedingungen Suspensionskulturen von grampositiven Bakterien und Dermatophyton in Gegenwart von reihenverdünnten Prüfsubstanzen angelegt. Das bakterielle Wachstum wird anhand der Trübung der Kulturen verfolgt und es wird die minimale Hemmkonzentration (MHK) und die minimale biozide Konzentration (MBK) ermittelt. Tabelle 2 zeigt die in diesem Test ermittelten Ergebnisse:
(Tabelle 2 wird nachgeliefert.)

Die antiinflammatorische Wirksamkeit der Hydroxyalkancarbonsäure-Derivate kann mittels des Rattenohrtests ermittelt werden, der wie folgt durchgeführt wird.

An der Außenseite von Rattenohren wird durch topische Applikation von 50 µl einer 5 %igen Lösung von Crotonöl in Ethanol ein Ödem induziert. Gleichzeitig wird eine 5 %ige Lösung der Prüfsubstanzen in Ethanol ebenfalls topisch appliziert 5 Stunden nach der Applikation wird die Hemmung der Gewichtszunahme des behandelten Ohres ermittelt, welche ein Maß für die antiinflammatorische Wirksamkeit der Verbindungen ist. Tabelle 3 zeigt die in diesen Test erhaltenen Ergebnisse.

**Tabelle 3**

| Nr. | Substanz | % Hemmung* |
|---|---|---|
| I | 11-Hydroxy-undecansäure-dimethylamid | 54 |
| II | 12-Hydroxy-dodecansäure-dimethylamid | 31 |
| III | 13-Hydroxy-tridecansäure-dimethylamid | 64 |
| IV | 14-Hydroxy-tetradecansäure-dimethylamid | 55 |

| | | |
|---|---|---|
| * bezogen auf die Vehikel-Kontrolle | | |

Zur Ermittlung der akuten Toxizität der erfindungsgemäßen Substanzen werden weißen Mäusen 100 mg/kg 13-Hydroxy-tridecansäure-dimethylamid intraperitoneal appliziert. 6 Stunden, 24 Stunden und 7 Tage nach der Applikation hatte sich der Allgemeinzustand und das Differentialblutbild der Versuchstiere nicht auffällig verändert.

Im Rahmen der bislang durchgeführten pharmakologischen Untersuchungen wurden bislang überwiegend solche Hydroxyalkancarbonsäure-Derivate der allgemeinen Formel I untersucht, die eine freie Hydroxygruppe tragen. Es ist aber sehr wahrscheinlich, daß alle Verbindungen mit einer veresterten Hydroxygruppe in vivo das gleiche Wirkungsspektrum zeigen werden, da diese Ester sehr wahrscheinlich bei der Penetration durch die Haut hydrolytisch gespalten werden. Die Hydrolysegeschwindigkeit der Ester und die Lipophilität dieser Verbindungen (und somit deren Penetrationsfähigkeit) wird bei den verschiedenen Estern unterschiedlich sein, wodurch es möglich sein wird, die Wirkungsintensität und Wirkungsdauer der Hydroxyalkancarbonsäure-Derivate gezielt zu verändern.

Als Acylgruppen der Acyloxyalkancarbonsäure-Derivate der allgemeinen Formel I gemäß Patentanspruch 1 eignen sich solche, die 1 bis 16, - vorzugsweise 1 bis 8 Kohlenstoffatome - besitzen.

Solche Acylgruppen sind beispielsweise geradkettige oder verzweigte Alkanoylgruppen, Cycloalkylcarbonylgruppen, Cycloalkylalkanoylgruppen, Benzoylgruppen, Alkylbenzoylgruppen und Phenylalkanoylgruppen die in üblicher Weise beispielsweise durch Hydroxygruppen. Alkoxygruppen (Methoxygruppen etc.) Alkanoyloxygruppen (Acetoxygruppen etc.), Aminogruppen, Halogenatome oder Carboxylgruppen substituiert sein können. Als geeignete Acylgruppen seien beispielsweise genannt: Die Formylgruppe, die Acetylgruppe, die Propionylgruppe, die Butyrylgruppe, die 2-Methylpropionylgruppe, die Pentanoylgruppe, die 2,2-Dimethylpropionylgruppe, die Hexanoylgruppe, die Cyclopentylcarbonylgruppe, die 3-Cyclopropylpropionylgruppe, die Octanoylgruppe, die Dodecanoylgruppe, die Bezoylgruppe, die Hydroxyacetylgruppe, die Methoxyacetylgruppe, die Acetoxyacetylgruppe, die Aminoacetylgruppe, die 3-Carboxypropionylgruppe, die 2-Hydroxybenzoylgruppe, die 2-Acetoxybenzoylgruppe, die 4-Methoxyacetylgruppe, die 4-Chlorbenzoylgruppe, die 4-Methylbenzoylgruppe und die Phenylacetylgruppe.

Aufgrund der bisher durchgeführten Untersuchungen und allgemeiner Erfahrungsgrundsätze kann man davon ausgehen, daß nicht nur die bereits getesteten Hydroxyalkancarbonsäure-Derivate und deren Ester das aufgezeigte Wirkungsspektrum besitzen, sondern daß auch Strukturanaloge dieser Substanzen eine vergleichbare Wirksamkeit zeigen. Derartige Strukturanaloge sind Hydroxyalkancarbonsäure-Derivate der allgemeinen Formel I gemäß Patentanspruch 1 mit n in der Bedeutung von 7,8, 17,18 oder insbesondere 9 und 10, 15 und 16, Hydroxyalkancarbonsäure-Derivate der allgemeinen Formel I gemäß Patentanspruch 1 mit R₂ und/oder R₃ (die unterschiedlich sein können) in der Bedeutung einer Alkylgruppe mit 2 bis 8 (vorzugsweise 2 bis 4) Kohlenstoffatomen, wie die Ethylgruppe, Propylgruppe, 2-Propylgruppe sowie Hydroxyalkancarbonsäure-Derivate der allgemeinen Formel I gemäß Patentanspruch 1 mit R₂ und R₃ in der Bedeutung einer gegebenenfalls durch ein Sauerstoffatom oder ein Stickstoffatom unterbrochene Alkylengruppe mit 4 bis 8 Kohlenstoffatomen. Derartige Alkylengruppen sind vorzugsweise Gruppierungen, welche gemeinsam mit dem Amidstickstoff ein Ringsystem der Teilformel VI bilden: 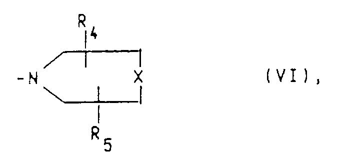
worin
R₄ und R₅ unabhängig voneinander ein Wasserstoffatom oder eine Methylgruppe darstellen und
X eine Kohlenstoff-Kohlenstoffbindung, eine Methylengruppe, ein Sauerstoffatom, eine NH-Gruppe oder eine 〉N-CH₃ Gruppe symbolisiert, bilden.

Derartige Ringsysteme sind beispielsweise der Pyrrolidinring, der Piperidinring, der Piperazinring und der Morpholinring sowie deren Methyl- oder Dimethylsubstituenten tragende Derivate.

Die Hydroxyalkancarbonsäure-Derivate der allgemeinen Formel können beispielsweise nach Verfahren hergestellt werden, welche unter Bedingungen durchgeführt werden, die dem Fachmann wohl bekannt sind.

Es wurde bereits eingangs ausführlich dargelegt, daß ein wesentlicher Aspekt der vorliegenden Erfindung die Verwendung der Hydroxyalkancarbonsäure-Derivate der allgemeinen Formel I zur Herstellung eines Arzneimittels für die lokale Behandlung von Erkrankungen der Haut und Schleimhaut ist.

Die Herstellung derartiger topisch zu applizierbaren Arzneimittel ist an sich vorbekannt. Andererseits ist es aber auch möglich, neue, den speziellen Bedürfnissen der Haut angepaßte Zubereitungen zu erstellen.

Die Herstellung solcher topischen Zubereitungen erfolgt in üblicher Weise, indem man die Wirkstoffe mit geeigneten Zusätzen in die gewünschte Applikationsform, wie zum Beispiel eine Lösung, eine Milch, eine Lotion, eine Creme, eine Salbe, oder eine Paste überfuhrt. In der so formulierten Zubereitung ist die Wirkstoffkonzentration von der Applikationsform abhängig. Vorzugsweise wird eine Wirkstoffkonzentration von 5 bis 30 Gewichtsprozent verwendet.

Die Milch, Lotion oder Creme (Öl/Wasser-Emulsionen) und die Salbe (Wasser/Öl-Emulsionen) kann in konventioneller Weise unter Verwendung konventioneller Emulgatoren hergestellt werden (Kirk Othmer: Enzyclopedia of Chemical Technology, 3. Auflage, 1979; John Wiley & Sons, New York, Vol. 8, Seiten 900 - 930, und Dr. Otto-Albrecht Neumüller: Römpps Chemie Lexikon, 7. Auflage, 1973; Franckh'sche Verlagshandlung Stuttgart, Seiten 1009 - 1013). Die für diese Emulsionen verwendeten Wachse, Emulgatoren und übrigen Zusätze sind die gleichen, wie man sie konventioneller Weise verwendet (Dr. Otto-Albrecht Neumüller: Römpps Chemie Lexikon, 7. Auflage, 1973; Franckh'sche Verlagshandlung Stuttgart, Seiten 1427 und 1428).

Die erfindungsgemäße topische Zubereitung kann aus einem oder zwei Wirkstoffen hydrophilen und/oder lipophilen Zusätzen, Fettphase, Öl/Wasser-Emulgator, wäßriger Phase und Konservierungsmittel bestehen.

Als hydrophile und/oder lipophile Zusätze können Feuchthaltefaktoren (Hydrokomplexe), wie zum Beispiel Propylenglykol, Glycerin, Polyäthylenglykole, Vitalkomplexe (wie zum Beispiel Placentaextrakte), Enzyme, Kräuterauszüge (wie zum Beispiel Hammamelis Extrakt oder Kamillenextrakt) oder Proteine (wie zum Beispiel Collagen) eingesetzt werden. Als ölige Phase oder als Fettphase in der Öl/Wasser-Emulsion eignen sich Kohlenwasserstoffe, wie zum Beispiel Squalen, Vaseline, Paraffine oder Stearin, oder Wachse, wie zum Beispiel Bienenwachs oder tierische oder pflanzliche Öle, wie Olivenöl, Erdnußöl, feines Knochenöl, Mandelöl, Jojoba-Öl, Lanolin oder Sonnenblumenöl. Geeignete Öl/Wasser-Emulgatoren sind beispielsweise Stearylalkohol, Polyoxyäthylenstearate (wie zum Beispiel MYRJ®), Komplexemulgatoren (wie zum Beispiel Amphoterin®) und Sorbitanfettsäureester (wie zum Beispiel Tween 80®), Carboxyvinylpolymerisate (wie zum Beispiel Carbopol®), Fettalkohole wie zum Beispiel Cetylalkohol, Myristylalkohol oder Mischester (wie zum Beispiel Dehymuls®). Die wäßrige Phase kann zusätzlich noch Puffersubstanzen, wie zum Beispiel das Dinatriumsalz der Äthylendiamin-N,N,N',N'-tetraessigsäure und Konservierungsmittel, wie Benzoesäure, Chlorquinaldol, Parabene oder Benzalkoniumchlorid, enthalten.

Die Emulsion wird zusätzlich mit einem oder zwei Wirkstoff(en) und gegebenenfalls noch mit Duftstoffen, wie zum Beispiel diejenige der Crematest®-Serie, versetzt und bis zur gleichmäßigen Verteilung derselben gerührt.

Es ist oft vorteilhaft, den erfindungsgemäßen Mitteln zusätzlich noch ca. 1 bis 4 Gewichtsprozent einer keratolytisch wirksamen Substanz, wie zum Beispiel Salicylsäure oder Resorcin - bezogen auf das Gesamtgewicht des Mittels - zuzusetzen.

Die nachfolgenden Ausführungsbeispiele dienen zur näheren Erläuterung der Erfindung.

### A) Ausführungsbeispiele betreffend die Herstellung von neuen HydroxyalkancarbonsäureDerivaten.

### Beispiel 1

a) 25 g 11-Brom-undecanol werden in 200 ml Acetanhydrid 5 Stunden lang unter Rückfluß erhitzt. Dann destilliert man das überschüssige Acetanhydrid im Vakuum ab, fraktioniert den Rückstand im Hochvakuum und erhält 28,6 g 1-Acetoxy-11-bromundecan vom Siedepunkt 118-120° C bei 6,7 Pa.
b) Eine Suspension von 0,6 g Natriumhydrid und 3 g Malonsäure-ethylester-dimethylamid in 30 ml Dimethylformamid wird eine Stunde lang bei 22° C gerührt, mit 6,9 g 1-Acetoxy-11-bromundecan versetzt und 16 Stunden lang bei 50° C gerührt. Anschließend rührt man die Reaktionsmischung in 200 ml Eiswasser, extrahiert mit Essigsäureethylester, trocknet die organische Phase über Natriumsulfat und engt sie im Vakuum ein. Man erhält so 5,3 g 11-Acetoxyundecan-malonsäureethylester-dimethylamid als hellgelbes Öl.
c) 4,04 g des so erhaltenen Produkts werden in 21,8 ml 1n wässriger Natronlauge suspendiert und 5 Stunden lang unter Rückfluß erhitzt. Man läßt die Reaktionsmischung erkalten, säuert sie mit 10n wässriger Salzsäure an, saugt das abgeschiedene Produkt ab und erhält so 3,5 g 1-Hydroxyundecanmalonsäure-dimethylamid als Rohprodukt.
d) Das erhaltene Rohprodukt wird bis zur Beendigung der CO₂ Abspaltung auf 150° C erhitzt (ca. eine Stunde), dann läßt man die Mischung erkalten und kristallisiert das erhaltene Rohprodukt aus Ethylacetat um. Man erhält so 1,92 g 13-Hydroxy-tridecansäure-dimethylamid vom Schmelzpunkt 69-71° C.

### Beispiel 2

a) 200 g Tridecan-1,13-disäure-dimethylester werden in 200 ml Methanol gelöst, mit einer Lösung von 116 g Bariumhydroxid-Octahydrat in 1,5 l Methanol versetzt und 38 Stunden lang bei 22° C gerührt. Dann saugt man den Niederschlag ab, suspendiert ihn in 3 Liter Eis-Wasser und säuert ihn mit 75 ml 10n wässriger Salzsäure an. Man extrahiert mit Essigsäureethylester, wäscht die organische Phase mit Wasser, trocknet sie über Natriumsulfat, engt sie im Vakuum ein und erhält 179 g Tridecan-1,13-disäure-monomethylester vom Schmelzpunkt 50-51° C.
b) Man gibt 26,3 ml Thionylchlorid zu 62,4 g Tridecan-1,13-disäure-monomethylester, erhitzt 2 Stunden lang auf 80° C und engt die Reaktionsmischung im Vakuum ein. Der Rückstand wird in 50 ml Diethylether gelost und innerhalb von 10 Minuten unter Eiskühlung zu einer Suspension von 67,3 ml einer 40 %igen wässrigen Dimethylaminlösung und 65 ml Diethylether zugetropft. Man rührt die Mischung in Eiswasser, extrahiert mit Essigsäureethylester, wäscht die organische Phase mit Wasser, trocknet sie über Natriumsulfat und engt sie im Vakuum ein. Das erhaltene Rohprodukt wird aus Hexan umkristallisiert und man erhält 38,3 g Tridecan-1,13-disäure-methylester-dimethylamid vom Schmelzpunkt 39-40° C.
c) 10 g Tridecan-1,13-disäure-methylester-dimethylamid werden in 140 ml tert.-Butanol gelost, mit 13,1 g Natriumborhydrid versetzt und auf 80° C erhitzt. Innerhalb von 45 Minuten werden 28 ml Methanol bei 80° C zugetropft. Dann läßt man die Mischung erkalten, versetzt sie mit 350 ml Eiswasser und extrahiert mit Essigsäureethylester, wäscht die organische Phase, trocknet sie über Natriumsulfat und engt sie im Vakuum ein. Das erhaltene Rohprodukt wird aus Cyclohexan-Essigsäureethylester umkristallisiert und man erhält 6,4 g 13-Hydroxytridecansäure-dimethylamid vom Schmelzpunkt 68-70° C.

### Beispiel 3

Unter den Bedingungen des Beispiels 2, aber ausgehend von dem Undecan-1,11-disäure-dimethylester wird das 11-Hydroxyundecansäure-dimethylamid hergestellt.

### Beispiel 4

Unter den Bedingungen des Beispiels 2, aber ausgehend von dem Dodecan-1,12-disäure-dimethylester wird das 12-Hydroxydodecansäure-dimethylamid hergestellt.

### Beispiel 5

Unter den Bedingungen des Beispiels 2, aber ausgehend von dem Tetradecan-1,14-disäure-dimethylester wird das 14-Hydroxy-tetradecansäure-dimethylamid hergestellt.

### Beispiel 6

15,0 g 13-Hydroxy-tridecansäure-dimethylamid werden mit 64,7 ml Essigsäureanhydrid und 80,4 ml Pyridin versetzt und 16 Stunden lang bei Raumtemperatur gerührt. Dann gießt man die Mischung auf Eiswasser, extrahiert mit Essigsäureethylester, wäscht die organische Phase, trocknet sie über Natriumsulfat und engt sie im Vakuum ein. Das gebildete Rohprodukt wird aus Hexan umkristallisiert und man erhält 13,27 g 13-Acetoxy-tridecansäure-dimethylamid vom Schmelzpunkt 44-45,5° C.

### Beispiel 7

Zu einer Mischung von 15,0 g 13-Hydroxytridecansäure-dimethylamid und 80 ml Pyridin werden unter Eiskühlung 84,2 ml 2, 2-Dimethylpropionsäurechlorid getropft. Man rührt die Reaktionsmischung 14 Stunden lang bei Raumtemperatur und gießt sie auf Eiswasser. Nach Zugabe von 70 g Natriumhydrogencarbonat extrahiert man mit Essigsäureethylester, wäscht die organische Phase, trocknet sie über Natriumsulfat und engt sie im Vakuum ein. Das erhaltene Rohprodukt wird über eine Kieselgelsäule (8x30 cm) mit Essigsäureethylester/Hexan (1+1) chromatographiert und man erhält 13,6 g 13-(2,2-Dimethylpropionyloxy)-tridecansäure-dimethylamid vom Schmelzpunkt 22° C.

### Beispiel 8

a) 176,1 g Tridecan-1,13-disäuremonomethylester werden mit 74,2 ml Thionylchlorid und 5,6 ml Dimethylformamid versetzt und 90 Minuten lang bei 80° C gerührt. Dann engt man die Mischung im Vakuum ein und erhält 191 g kristallinen Tridecan-1,13-disäurechlorid-methylester als Rohprodukt.
b) Zu einer Losung von 25,0 g Tridecan-1,13-disaurechlorid-methylester-Rohprodukt in 100 ml Diethylether tropft man bei -10° C eine Lösung von 27,21 ml Dipropylamin in 25 ml Diethylether. Dann setzt man der Mischung noch 60 ml Diethylether zu und rührt sie noch 150 Minuten lang bei 0° C. Die Reaktionsmischung wird in ein Gemisch von Eis/verdünnter Salzsäure gegossen und mit Diethylether extrahiert. Die organische Phase wird gewaschen, über Natriumsulfat getrocknet, im Vakuum eingeengt und man erhält 29,34 g Tridecan-1,13-disäure-methylester-dipropylamid als Rohprodukt.
c) Zu einer siedenen Suspension von 29,34 g Tridecan-1,13-disäure-methylesterdipropylamid in 400 ml tert.-Butanol und 32,2 g Natriumborhydrid tropft man innerhalb von 45 Minuten unter Inertgas 68,8 ml Methanol. Dann erhitzt man die Reaktionsmischung noch 100 Minuten lang unter Rückfluß, läßt sie erkalten und gießt sie in eine Mischung aus Eis, Wasser und Natriumchlorid. Man extrahiert mit Essigsäureethylester, wäscht die organische Phase mit gesättigter wässriger Natriumchlorid-Losung, trocknet sie über Natriumsulfat und engt sie im Vakuum ein. Der Rückstand wird über eine Kieselgelsäule (7x35 cm) mit Essigsäureethylester/Hexan (1+1) chromatographiert und man erhält 14,3 g 13-Hydroxytridecansäure-dipropylamid vom Schmelzpunkt 33,5-35,0° C.

### Beispiel 9

a) Unter den Bedingungen des Beispiels 8b werden 25,0 g Tridecan-1,13-disäurechlorid-methylester mit 16,6 ml Pyrrolidin umgesetzt, aufbereitet und man erhält 24,32 g Tridecan-1,13-disäure-methylester-tetramethylenamid vom Schmelzpunkt 41-42° C.
b) In eine siedende Suspension von 24,32 g Tridecan-1,13-disäure-methylestertetramethylenamid in 350 ml tert.-Butanol und 29,28 g Natriumborhydrid werden innerhalb von 30 Minuten 62,6 ml Methanol eingetropft. Man erhitzt die Mischung noch 150 Minuten lang unter Rückfluß und arbeitet sie auf wie in Beispiel 8c beschrieben. Das so erhaltene Rohprodukt wird aus Cyclohexan/Essigsäureethylester umkristallisiert und ergibt 13,53 g 13-Hydroxytridecansäure-tetramethylenamid vom Schmelzpunkt 60-61° C.

### Beispiel 10

a) Unter den Bedingungen des Beispiels 8b werden 25,0 g Tridecan-1,13-disäurechlorid-methylester mit 15,8 ml 2,6-Dimethylmorpholin umgesetzt und aufbereitet. Das erhaltene Roprodukt wird über eine Kieselgelsäule (5x100 cm) mittels Hexan-Essigsäureethylester/Hexan (1+2) Gradienten chromatographiert und man erhält 10,4 g Tridecan-1,13-disäure-methylester-(2,4-dimethyl-3-oxapentamethylen)amid als Öl.
b) Zu einer siedenden Suspension von 10,2 g Tridecan-1,13-disäure-methylester-(2,4-dimethyl-3-oxapentamethylen)amid in 150 ml tert.-Butanol und 12,28 g Natriumborhydrid wurden innerhalb von 20 Minuten 26,27 ml Methanol zugetropft. Dann erhitzt man die Mischung noch 150 Minuten lang unter Rückfluß, arbeitet sie auf wie in Beispiel 8c beschrieben, kristallisiert das erhaltene Rohprodukt aus Cyclohexan/Essigsäureethylester um und erhält 5,58 g 13-Hydroxy-tridecansäure-(2,4-dimethyl-3-oxapentamethylen)-amid vom Schmelzpunkt 86-87,5° C.

### B) Ausführungsbeispiel betreffend eine galenische Zubereitung

| Zusammensetzung | |
|---|---|
| | Gewichtsprozent |
| 13-Hydroxy-tridecansäure-dimethylamid | 20,0 |
| Benzoesäure | 0,1 |
| Salicylsäure | 2,0 |
| Glycerinmonostearat | 2,0 |
| Cetylalkohol | 3,0 |
| Polyoxyethylen(20)sorbitanmonooleat | 5,0 |
| Natrium-laurylethersulfat | 10,0 |
| Ethanolamin-laurylethersulfat | 1,0 |
| Olivenöl | 2,0 |
| Ascorbinsäure | 1,0 |
| Bidestilliertes Wasser | 53,9 |

### Zubereitung

13-Hydroxy-tridecansäure-dimethylamid, Benzoesäure, Polyoxyethylen(20)sorbitanmonolat, Natrium-laurylethersulfat, Ethanolamin-laurylethersulfat und Wasser werden auf 60° C erwärmt und mit der Mischung von Salicylsäure, Glycerinmonostearat, Cetylalkohol, Olivenöl und Ascorbinsäure bei 40° C dispergiert.

## Patentansprüche

1. Verwendung von Hydroxyalkancarbonsäure-Derivaten der allgemeinen Formel I 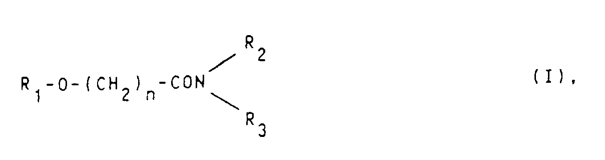 worin
n die Ziffern 7 bis 18 bedeutet,
R₁ ein Wasserstoffatom oder eine Acylgruppe mit maximal 16 Kohlenstoffatomen darstellt und
R₂ und R₃ jeweils Alkylgruppen mit maximal 8 Kohlenstoffatomen oder gemeinsam eine gegebenenfalls durch ein Sauerstoffatom oder ein Stickstoffatom unterbrochene Alkylengruppe mit 4 bis 8 Kohlenstoffatomen bedeutet, zur Herstellung eines Arzneimittels für die lokale Behandlung von Erkrankungen der Haut und Schleimhaut.

2. 11-Hydroxy-undecansäure-diemethylamid.

3. 12-Hydroxy-dodecansäure-diemethylamid.

4. 13-Hydroxy-tridecansäure-dimethylamid.

5. 14-Hydroxy-tetracansäure-dimethylamid.

6. 13-Acetoxy-tridecansäure-dimethylamid.

7. 13-(2,2-Dimethylpropionyloxy)-tridecansäure-dimethylamid.

8. 13-Hydroxy-tridecansäure-dipropylamid.

9. 13-Hydroxy-tridecansäure-tetramethylenamid.

10. 13-Hydroxy-tridecansäure-(2,4-dimethyl-3-oxa-pentamethylen)-amid.

## Claims

1. Use of hydroxyalkanecarboxylic acid derivatives of the general formula I 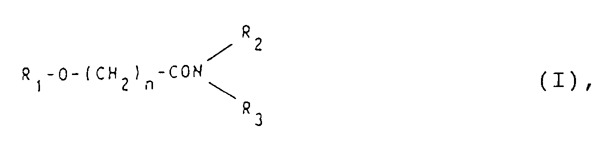 in which
n represents the numbers 7 to 18,
R₁ represents a hydrogen atom or an acyl group having not more than 16 carbon atoms, and
R₂ and R₃ each represent alkyl groups having not more than 8 carbon atoms or together represent an alkylene group having from 4 to 8 carbon atoms that is optionally interrupted by an oxygen atom or a nitrogen atom,
for the manufacture of a medicament for the local treatment of disorders of the skin and the mucous membrane.

2. 11-hydroxyundecanoic acid dimethylamide.

3. 12-hydroxydodecanoic acid dimethylamide.

4. 13-hydroxytridecanoic acid dimethylamide.

5. 14-hydroxytetradecanoic acid dimethylamide.

6. 13-acetoxytridecanoic acid dimethylamide.

7. 13-(2,2-dimethylpropionyloxy)-tridecanoic acid dimethylamide.

8. 13-hydroxytridecanoic acid dipropylamide.

9. 13-hydroxytridecanoic acid tetramethyleneamide.

10. 13-hydroxytridecanoic acid (2,4-dimethyl-3-oxa-pentamethylene)-amide.

## Revendications

1. Utilisation de dérivés d'acides hydroxyalcane carboxyliques de formule générale I : 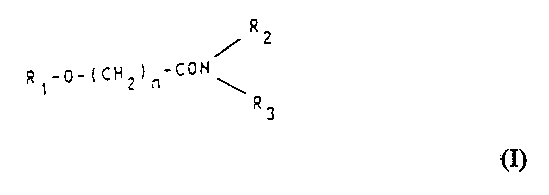 dans laquelle
n représente les nombres 1 à 18;
R¹ représente un atome d'hydrogène ou un groupe acyle ayant au maximum 16 atomes de carbone ;
R² et R³ représentent chacun un groupe alkyle ayant au maximum 8 atomes de carbone ou forment ensemble un groupe alkylène ayant 4 à 8 atomes de carbone et qui est éventuellement interrompu par un atome d'oxygène ou par un atome d'azote pour la production d'un médicament destiné au traitement local de maladies de la peau et des muqueuses.

2. Le diméthylamide de l'acide 11-hydroxy-undécanoïque.

3. Le diméthylamide de l'acide 12-hydroxy-dodécanoïque.

4. Le diméthylamide de l'acide 13-hydroxy-tridécanoïque.

5. Le diméthylamide de l'acide 14-hydroxy-tétradécanoïque.

6. Le diméthylamide de l'acide 13-acétoxy-tridécanoïque.

7. Le diméthylamide de l'acide 13-(2,2-diméthylpropionyloxy)-tridécanoïque.

8. Le dipropylamide de l'acide 13-hydroxy-tridécanoïque.

9. Le tétraméthylène-amide de l'acide 13-hydroxy-tridécanoïque.

10. Le (2,4-diméthyl-3-oxa-pentaméthylène)-amide de l'acide 13-hydroxy-tridécanoïque.
